# EUROPÄISCHE PATENTSCHRIFT

(11) **EP 3 873 424 B1**
(45) Veröffentlichungstag und Bekanntmachung des Hinweises auf die Patenterteilung: **09.04.2025**
(21) Anmeldenummer: 19797682.2
(22) Anmeldetag: 31.10.2019
(51) Int. Cl.: A61K 8/58, A61K 8/81, A61Q 5/00, A61Q 5/12, A61Q 17/00, A61Q 5/06

(54) **WIRKSTOFFZUSAMMENSETZUNG ZUR FORMVERÄNDERUNG DES HAARKOLLEKTIVES**
ACTIVE INGREDIENT COMPOSITION FOR CHANGING THE SHAPE OF THE HAIR COLLECTIVE
COMPOSITION DE PRINCIPE ACTIF POUR CHANGER LA FORME DU COLLECTIF DE CHEVEUX

(30) Priorität: 31.10.2018 DE 102018127186
(43) Veröffentlichungstag der Anmeldung: 08.09.2021
(73) Patentinhaber: Henkel AG & Co. KGaA, 40589 Düsseldorf (DE)
(72) Erfinder: KROHN, Rene, 22848 Norderstedt (DE); SCHULZE ZUR WIESCHE, Erik, 33619 Bielefeld (DE)
(86) Internationale Anmeldenummer: PCT/EP2019/079781
(87) Internationale Veröffentlichungsnummer: WO 2020/089367

(56) Entgegenhaltungen:
- WO-A1-2017/029336
- WO-A1-2017/102856
- WO-A2-2012/175681
- US-A1- 2002 037 298
- US-A1- 2007 275 020
- CHEMICAL ABSTRACTS, Columbus, Ohio, US; abstract no. 18784-74-2
- DATABASE MINTEL 1 September 2018 (2018-09-01), ANONYMOUS: "Mega Strong Hairspray"
- DATABASE GNPD [online] MINTEL; 14 November 2016 (2016-11-14), ANONYMOUS: "Be Merry & Bright Fullfyl Holiday Gift Set", XP055651473, retrieved from https://www.gnpd.com/sinatra/recordpage/4405807/ Database accession no. 4405807
- DATABASE GNPD [online] MINTEL; 14 November 2016 (2016-11-14), ANONYMOUS: "Be Merry & Bright Fullfyl Holiday Gift Set", XP055651473, retrieved from www.gnpd.com Database accession no. 4405807

## Beschreibung

Die Offenbarung betrifft kosmetische Mittel zur Behandlung eines keratinischen Materials, wobei das Mittel eine organische Siliciumverbindung und mindestens ein Copolymer umfasst, welches zumindest aus den Monomereinheiten N-tert-Octylacrylamid, Acrylsäure und tert.-Butylaminoethylmethacrylat aufgebaut ist, sowie die Verwendung des kosmetischen Mittels.

Die äußere Beanspruchung der Haare durch chemische Stoffe aus einer Vielzahl unterschiedlicher Quellen stellt die Entwicklung kosmetischer Produkte vor Herausforderungen. Luft- und Wasserverunreinigungen wirken sich nachteilig auf Haut und Haare aus. Zu den wichtigsten Luftschadstoffen gehören polycyclische aromatische Kohlenwasserstoffe, flüchtige organische Verbindungen, Stickoxide (NOx), Partikel und Zigarettenrauch. Die Wirkung verschiedener Luftschadstoffe kann in Gegenwart anderer Luftschadstoffe und unter Einwirkung von UV-Strahlung oder Wärme verstärkt werden.

Es ist bekannt, dass die Toxizität von gasförmigen Schadstoffen der Luft, wie Schwefeldioxid, Ozon und Stickoxiden, insbesondere mit ihrer Initiatoraktivität für freie Radikale zusammenhängt, die bei Lebewesen Schäden verursachen. Freie Radikale sind Stoffwechselprodukte, die auch natürlicherweise im Körper vorkommen. In grosser Menge können freie Radikale Irritationen und Entzündungen begünstigen und den Prozess der Alterung beschleunigen. In dem Fall spricht man von "oxidativem Schaden". Freie Radikale können auch eine Haarschädigung bewirken, die beispielsweise als Verringerung des Glanzes sowie des Griffs und/oder des Verblassens der Haarfarbe sichtbar wird. Hitzeeinwirkung verstärkt natürlich die Schädigung, die durch Schadstoffe verursacht wird.

Kundenwünsche betreffen aber nicht nur Merkmale, die das Erscheinungsbild der Haare an sich betreffen. Das Anforderungsprofil an kosmetische Mittel für die Haare ist vielfältiger. Kosmetische Mittel, die einer permanenten oder temporären Formgebung der Haare dienen, spielen in der Kosmetik ebenfalls eine wichtige Rolle. So sollen kosmetische Mittel eine temporäre Formgebung mit einem guten Frisurenhalt ermöglichen. Die Entwicklung von kosmetischen Mitteln, die einen guten Frisurenhalt ermöglichen und eine Schutzfunktion gegenüber Schädigung durch Schadstoffe ermöglichen, stellt eine Herausforderung dar.

Im Stand der Technik werden siliciumorganische Verbindungen aus der Gruppe der Silane beschrieben, die mindestens eine Hydroxygruppe und/oder hydrolysierbare Gruppe umfassen. Aufgrund der Anwesenheit der Hydroxygruppen und/oder hydrolysierbaren Gruppen handelt es sich bei den Silanen um reaktive Substanzen, die in Gegenwart von Wasser hydrolysieren bzw. oligomerisieren oder polymerisieren. Die durch Anwesenheit des Wassers initiierte Oligomerisierung oder Polymerisierung der Silane führt bei Anwendung auf einem keratinischen Material letztendlich zur Ausbildung eines Films, der eine Schutzwirkung entfalten kann.

Die der vorliegenden Erfindung zugrunde liegende Aufgabe besteht in der Bereitstellung eines kosmetischen Produkts, das eine gute Formgebung der Haare ermöglicht und den Strukturschutz gegenüber Hitzeeinwirkung verbessert.

Diese Aufgabe wird gelöst durch ein kosmetisches Mittel zur Behandlung eines keratinischen Materials It. Anspruch 1.

Unter einem keratinischen Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter einem keratinischen Material das menschliche Haar, die menschliche Haut und menschliche Nägel, insbesondere Finger- und Fußnägel, verstanden. Ganz besonders bevorzugt wird unter keratinischem Material das menschliche Haar, insbesondere Kopf- und/oder Barthaare, verstanden.

Als ersten wesentlichen Bestandteil der Offenbarung enthält das kosmetische Mittel zur Behandlung eines keratinischen Materials mindestens eine organische Siliciumverbindung. Bevorzugte organische Siliciumverbindungen werden ausgewählt aus Silanen mit einem, zwei oder drei Siliciumatomen, wobei die organische Siliciumverbindung eine oder mehrere Hydroxylgruppen und/oder hydrolysierbare Gruppen pro Molekül umfasst.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die organische Siliciumverbindungen sind Verbindungen, die ein bis drei Siliciumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die WasserstoffAtome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Das offenbarte Mittel zur Behandlung eines keratinischen Materials enthält mindestens eine organischen Siliciumverbindung, die bevorzugt aus Silanen mit einem, zwei oder drei Siliciumatomen ausgewählt ist, wobei die organische Siliciumverbindung eine oder mehrere Hydroxylgruppen oder hydrolysierbare Gruppen pro Molekül umfasst.

Im Rahmen dieser Offenbarung weist das Mittel zur Behandlung eines keratinischen Materials mindestens eine organische Siliciumverbindung auf, die aus Silanen mit einem, zwei oder drei Siliciumatomen ausgewählt ist, wobei die organische Siliciumverbindung außerdem eine oder mehrere basische Gruppe und eine oder mehrere Hydroxylgruppen oder hydrolysierbare Gruppen pro Molekül umfasst.

Bei dieser basischen Gruppe kann es sich beispielsweise um eine Aminogruppe, eine Alkylaminogruppe oder um eine Dialkylaminogruppe handeln, die bevorzugt über einen Linker mit einem Siliciumatom verbunden ist. Bevorzugt handelt es sich bei der basischen Gruppe um eine Aminogruppe, eine C₁-C₆-Alkylaminogruppe oder um eine Di(C₁-C₆)alkylaminogruppe.

Bei der oder den hydrolysierbaren Gruppen handelt es sich bevorzugt um eine C₁-C₆-Alkoxygruppe, insbesondere um eine Ethoxygruppe oder um eine Methoxygruppe. Es ist bevorzugt, wenn die hydrolysierbare Gruppe direkt an das Siliciumatom gebunden vorliegt. Handelt es sich beispielsweise bei der hydrolysierbaren Gruppe um eine Ethoxygruppe, so enthält die organische Siliciumverbindung bevorzugt eine Struktureinheit R'R"R‴Si-O-CH₂-CH₃. Die Reste R', R" und R‴ stellen hierbei die drei übrigen freien Valenzen des Siliciumatoms dar.

Bevorzugt offenbart ist, dass das Mittel zur Behandlung eines keratinischen Materials mindestens eine organische Siliciumverbindung der Formel (I) und/oder (II) enthält.

Die Verbindungen der Formeln (I) und (II) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Das offenbarte Mittel zur Behandlung eines keratinischen Materials enthält mindestens eine organische Siliciumverbindung der Formel (I) oder (I) und (II),

R₁R₂N-L-Si(OR₃)ₐ(R₄)_{b} (I),

wobei
- R₁, R₂ beide für ein Wasserstoffatom stehen,
- L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht,
(R₅O)_{c}(R₆)_{d}Si-(A)ₑ-[NR₇-(A')]_{f}-[O-(A")]_{g}-[NR₈-(A‴)]ₕ-Si(R_{6'})_{d'}(OR₅')_{c'} (II),

wobei
- R5, R5', R5" unabhängig voneinander für ein Wasserstoffatom oder für eine C₁-C₆-Alkylgruppe stehen,
- R6, R6' und R6" unabhängig voneinander für eine C₁-C₆-Alkylgruppe stehen,
- A, A', A", A‴ und A"" unabhängig voneinander für eine lineare oder verzweigte, zweibindige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (III) stehen

- (A"")-Si(R₆")_{d}"(OR₅")_{c}" (III),

- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
- mit der Maßgabe, dass mindestens einer der Reste aus e, f, g und h von 0 verschieden ist.

Die Substituenten R₁, R₂, R₃, R₄, R₅, R₅', R₅", R₆, R₆', R₆", R₇, R₈, L, A, A', A", A‴ und A"" in den Verbindungen der Formel (I) und (II) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweibindige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweibindige Alkylengruppen auch verzweigt sein. Beispiele für verzweigte, zweibindige C₃-C₂₀-Alkylengruppen sind (-CH₂-CH(CH₃)-) und (-CH₂-CH(CH₃)-CH₂-).

In den organischen Siliciumverbindungen der Formel (I)

R₁R₂N-L-Si(OR₃)ₐ(R₄)_{b} (I),

stehen die Reste R₁ und R₂ unabhängig voneinander für ein Wasserstoffatom oder eine C₁-C₆-Alkylgruppe. Ganz besonders bevorzugt stehen die Reste R₁ und R₂ beide für ein Wasserstoffatom.

Im Mittelteil der organischen Siliciumverbindung befindet sich die Struktureinheit oder der Linker -L- der für eine lineare oder verzweigte, zweibindige C₁-C₂₀-Alkylengruppe steht.

Bevorzugt steht -L- für eine lineare, zweibindige C₁-C₂₀-Alkylengruppe. Weiter bevorzugt steht -L- für eine lineare zweibindige C₁-C₆-Alkylengruppe. Besonders bevorzugt steht -L- für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die organischen Siliciumverbindungen der Formel (I)

R₁R₂N-L-Si(OR₃)ₐ(R₄)_{b} (I),

tragen jeweils an einem Ende die Silicium-haltige Gruppierung -Si(OR₃)ₐ(R₄)_{b}.

In der endständigen Struktureinheit -Si(OR₃)ₐ(R₄)_{b} steht der Rest R₃ für ein Wasserstoffatom oder eine C₁-C₆-Alkylgruppe, und der Rest R₄ steht für eine C₁-C₆-Alkylgruppe. Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Der beste Schutz vor den negativen Auswirkungen von Wasser- und/oder Luftverschmutzungen ("Anti-Pollution"-Wirkung) und die beste Pflege von beanspruchtem Haar konnte erhalten werden, wenn das Mittel zur Behandlung eines keratinischen Materials mindestens eine organische Siliciumverbindung der Formel (I) enthält, in welcher die Reste R₃, R₄ unabhängig voneinander für eine Methylgruppe oder für eine Ethylgruppe stehen.

Im Detail offenbarte organische Siliciumverbindungen der Formel (I) sind
- (3-Aminopropyl)triethoxysilan 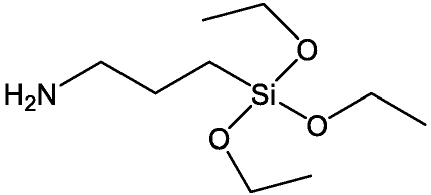
- (3-Aminopropyl)trimethoxysilan 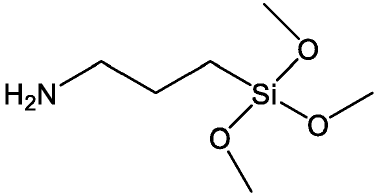
- 1-(3-Aminopropyl)silantriol 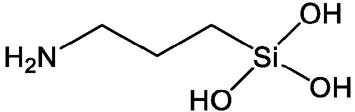
- (2-Aminoethyl)triethoxysilan 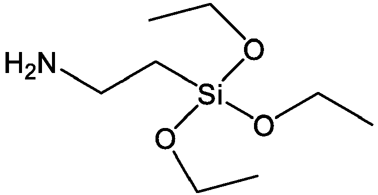
- (2-Aminoethyl)trimethoxysilan 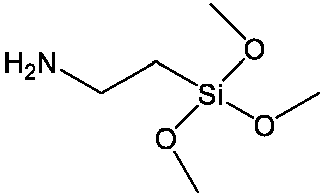
- 1-(2-Aminoethyl)silantriol 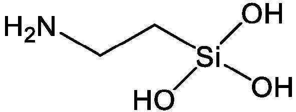
- (3-Dimethylaminopropyl)triethoxysilan 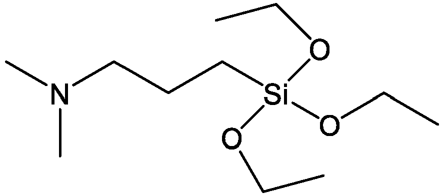
- (3-Dimethylaminopropyl)trimethoxysilan 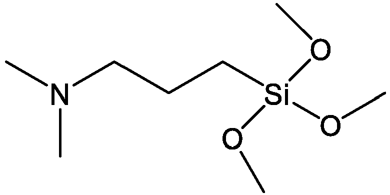
- 1-(3-Dimethylaminopropyl)silantriol 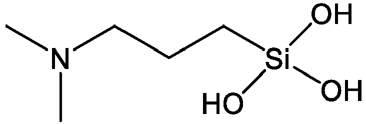
- (2-Dimethylaminoethyl)triethoxysilan. 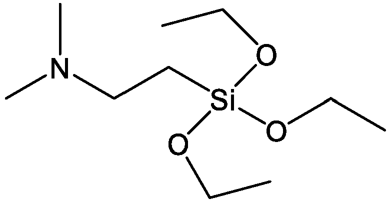
- (2-Dimethylaminoethyl)trimethoxysilan und/oder 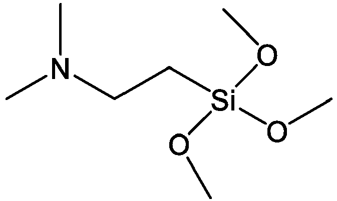
- 1-(2-Dimethylaminoethyl)silantriol

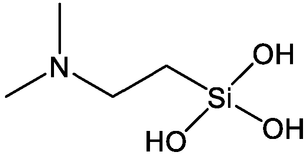

Die vorgenannten organische Siliciumverbindung der Formel (I) sind kommerziell erhältlich.

(3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erhältlich.

Im Rahmen einer weiteren Ausführungsform enthält das Mittel zur Behandlung eines keratinischen Materials zusätzlich ggf. eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II).

Die siliciumorganischen Verbindungen der Formel (II) tragen jeweils an ihren beiden Enden die Siliciumhaltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}- und -[O-(A")]_{g}- und -[NRₐ-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine organischen Siliciumverbindung der Formel (II) mindestens eine Gruppierung aus der Gruppe aus -(A)- und -[NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c'} stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3

steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Eine sehr hohe Anti-Pollution-Wirkung des Mittels zur Behandlung eines keratinischen Materials konnte erhalten werden, wenn die Reste c und c' beide für die Zahl 3 stehen. In diesem Fall stehen d und d' beide für die Zahl 0.

Weiter kann das Mittel zur Behandlung eines keratinischen Materials mindestens eine organische Siliciumverbindung der Formel (II) enthalten

(R₅O)_{c}(Rₑ)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die organischen Siliciumverbindung der Formel (IIa)

(R₅O)₃Si-(A)ₑ-[NR₇-(A')]_{f}-[O-(A")]_{g}-[NR₈-(A‴)]ₕ-Si(OR₅')₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NRₐ-(A‴)]ₕ- sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der "Anti-Pollution"-Wirkung erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die organischen Siliciumverbindung der Formel (IIb)

(R₅O)_{c}(R₆)_{d}Si-(A)-[NR₇-(A')]-Si(R₆')_{d'}(OR₅')_{c'} (IIb).

Die Reste A, A', A", A‴ und A"" stehen unabhängig voneinander für eine lineare oder verzweigte, zweibindige C₁-C₂₀-Alkylengruppe. Bevorzugt stehen die Reste A, A', A", A‴ und A"" unabhängig voneinander für eine lineare, zweibindige C₁-C₂₀-Alkylengruppe. Weiter bevorzugt stehen die Reste A, A', A", A‴ und A"" unabhängig voneinander für eine lineare zweibindige C₁-C₆-Alkylengruppe. Besonders bevorzugt stehen die Reste A, A', A", A‴ und A"" unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und A"" für eine Propylengruppe (-CH₂-CH₂-CH₂-).

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III)

- (A"")-Si(R₆")_{d}"(OR₅")_{c}" (III).

Ganz besonders bevorzugt stehen die Reste R₇ und R₈ unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die organische Siliciumverbindung die Gruppierung [NR₇-(A')], aber nicht die Gruppierung -[NR₈-(A‴)]. Steht nun der Rest R7 für eine Gruppierung der Formel (III), so enthält das Mittel zur Behandlung eines keratinischen Materials eine organische Siliciumverbindung mit 3 reaktiven Silan-Gruppen.

In einer weiteren Offenbarung enthält das Mittel zur Behandlung eines keratinischen Materials mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- e und f beide für die Zahl 1 stehen,
- g und h beide für die Zahl 0 stehen,
- A und A' unabhängig voneinander für eine lineare, zweibindige C₁-C₆-Alkylengruppe stehen
   und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform enthält das Mittel zur Behandlung eines keratinischen Materials mindestens eine organische Siliciumverbindung der Formel (II), wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂-) oder eine Propylengruppe (-CH₂-CH₂-CH₂) stehen,
   und
- R₇ für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der Aufgabenstellung gut geeignete organische Siliciumverbindungen der Formel (II) sind
- 3-(Trimethoxysilyl)-N-[3-(trimethoxysilyl)propyl]-1-propanamin 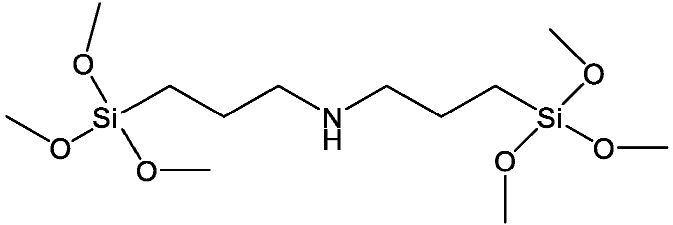
- 3-(Triethoxysilyl)-N-[3-(triethoxysilyl)propyl]-1-propanamin 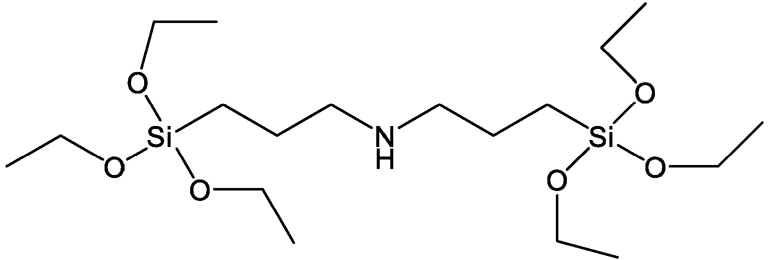
- N-Methyl-3-(trimethoxysilyl)-N-[3-(trimethoxysilyl)propyl]-1-propanamin 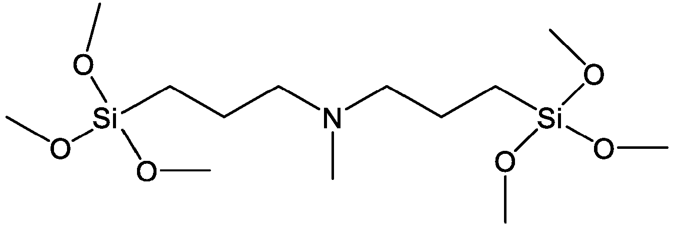
- N-Methyl-3-(triethoxysilyl)-N-[3-(triethoxysilyl)propyl]-1-propanamin 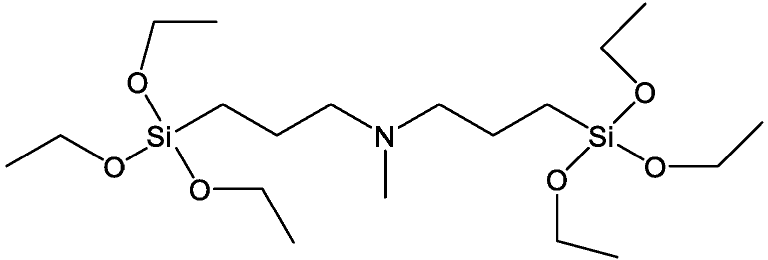
- 2-[Bis[3-(trimethoxysilyl)propyl]amino]-ethanol 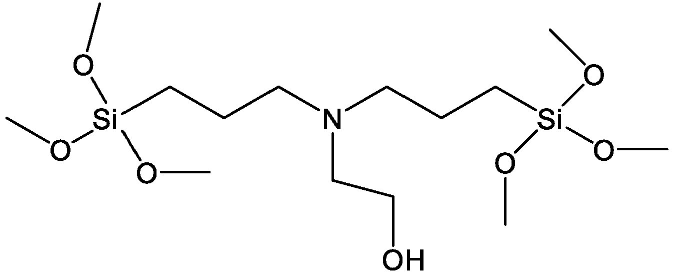
- 2-[Bis[3-(triethoxysilyl)propyl]amino]-ethanol 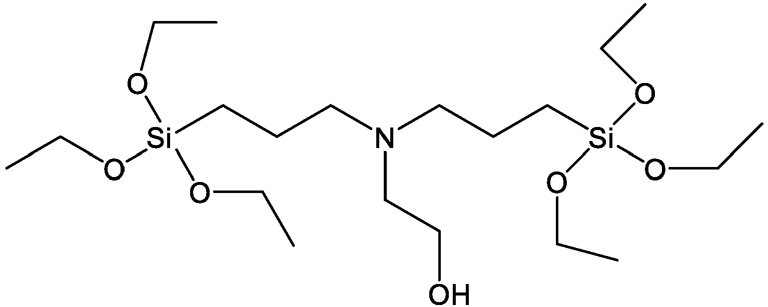
- 3-(Trimethoxysilyl)-N,N-bis[3-(trimethoxysilyl)propyl]-1-propanamin 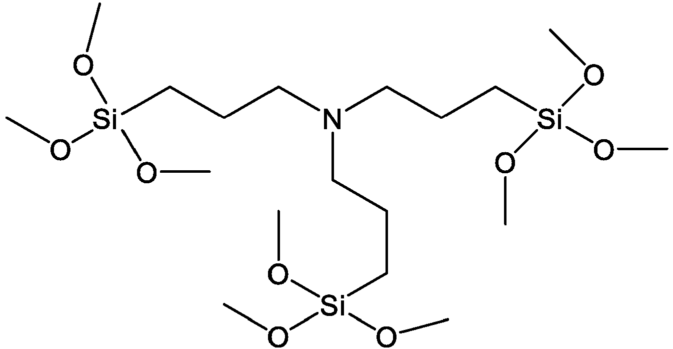
- 3-(Triethoxysilyl)-N,N-bis[3-(triethoxysilyl)propyl]-1-propanamin 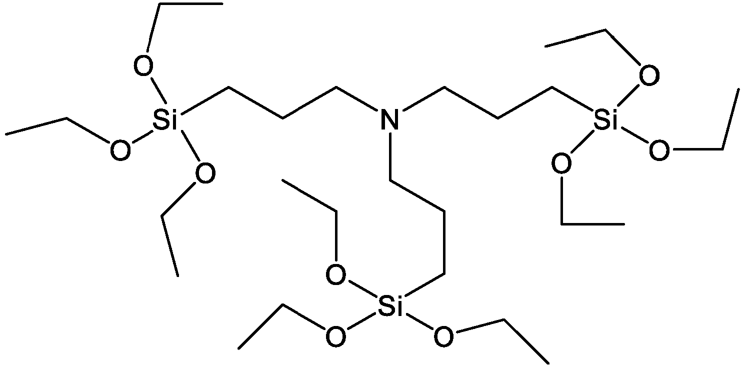
- N1,N1-Bis[3-(trimethoxysilyl)propyl]-1,2-ethanediamin, 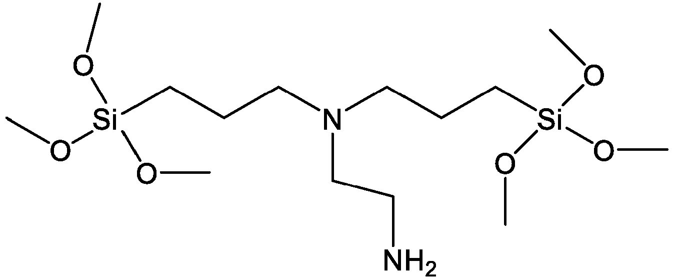
- N1,N1-Bis[3-(triethoxysilyl)propyl]-1,2-ethanediamin, 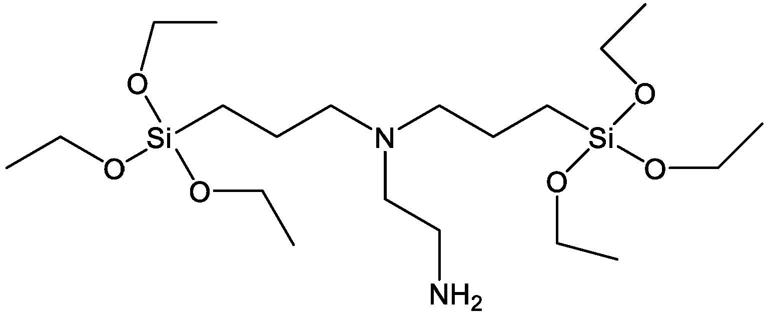
- N,N-Bis[3-(trimethoxysilyl)propyl]-2-propen-1-amin 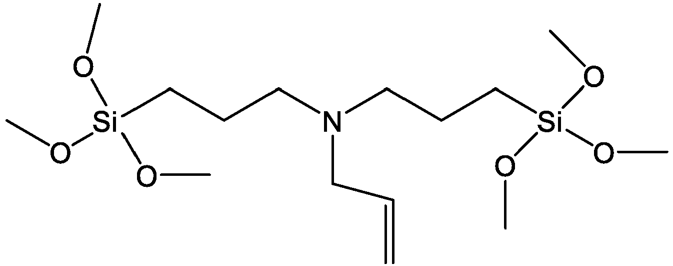
- N,N-Bis[3-(triethoxysilyl)propyl]-2-propen-1-amin

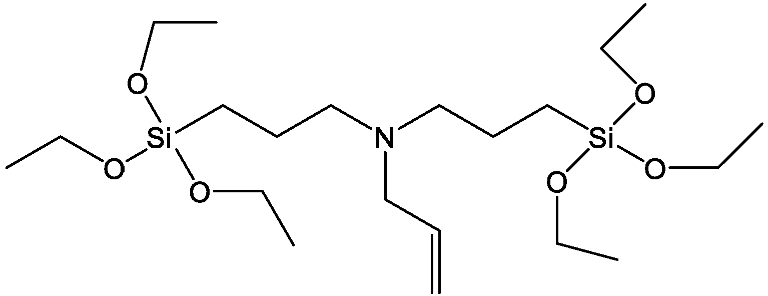

Die vorgenannten organische Siliciumverbindung der Formel (II) sind kommerziell erhältlich.

Bis(trimethoxysilylpropyl)amin mit der CAS-Nummer 82985-35-1 kann beispielsweise von Sigma-Aldrich käuflich erworben werden.

Bis[3-(triethoxysilyl)propyl]amin, auch bezeichnet als 3-(Triethoxysilyl)-N-[3-(triethoxysilyl)propyl]-1-propanamin, mit der CAS-Nummer 13497-18-2 kann zum Beispiel von Sigma-Aldrich käuflich erworben werden oder ist im Handel unter der Produktbezeichnung Dynasylan 1122 von Evonik erhältlich. N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

Ferner ist offenbart, dass das Mittel mindestens eine organische Siliciumverbindung der Formel (IV) enthält

R₉Si(OR₁₀)ₖ(R₁₁)ₘ (IV).

Die Verbindungen der Formel (IV) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Das bzw. die organischen Siliciumverbindungen der Formel (IV) können auch als Silane vom Typ der Alkylalkoxysilane oder der Alkylhydroxysilane bezeichnet werden,

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

Ferner offenbart enthält das Mittel zur Behandlung eines keratinischen Materials zusätzlich zu der oder den organischen Siliciumverbindungen der Formel (I) mindestens eine weitere organische Siliciumverbindung der Formel (IV)

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

Ferner offenbart das Mittel zur Behandlung eines keratinischen Materials zusätzlich zu der oder den organischen Siliciumverbindungen der Formel (II) mindestens eine weitere organische Siliciumverbindung der Formel (IV) enthält

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

Ferner offenbart enthält das Mittel zur Behandlung eines keratinischen Materials zusätzlich zu den organischen Siliciumverbindungen der Formel (I) und (II) mindestens eine weitere organische Siliciumverbindung der Formel (IV)

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In den organischen Siliciumverbindungen der Formel (IV) steht der Rest R₉ für eine C₁-C₁₂-Alkylgruppe.

Diese C₁-C₁₂-Alkylgruppe ist gesättigt und kann linear oder verzweigt sein. Bevorzugt steht R9 für eine lineare C₁-C₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht R₉ für eine Methylgruppe, eine Ethylgruppe oder eine n-Octylgruppe.

In den organischen Siliciumverbindungen der Formel (IV) steht der Rest R₁₀ für ein Wasserstoffatom oder eine C₁-C₆-Alkylgruppe. Besonders bevorzugt steht R₁₀ für eine Methylgruppe oder für eine Ethylgruppe.

In den organischen Siliciumverbindungen der Formel (IV) steht der Rest R₁₁ für eine C₁-C₆-Alkylgruppe.

Besonders bevorzugt steht R₁₁ für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Eine sehr hohe "Anti-Pollution"-Wirkung konnte erhalten werden, wenn das Mittel zur Behandlung eines keratinischen Materials mindestens eine organische Siliciumverbindung der Formel (IV) enthält, in welcher der Rest k für die Zahl 3 steht. In diesem Fall steht der Rest m für die Zahl 0.

Ferner speziell offenbart sind organische Siliciumverbindungen der Formel (IV) sind
- Methyltrimethoxysilan 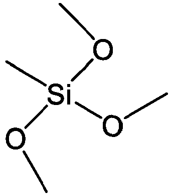
- Methyltriethoxysilan 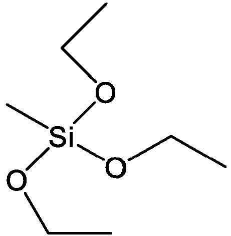
- Ethyltrimethoxysilan 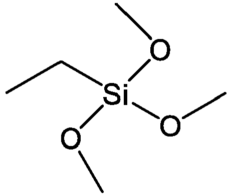
- Ethyltriethoxysilan 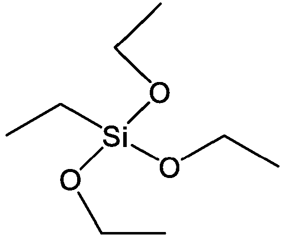
- n-Hexyltrimethoxysilan 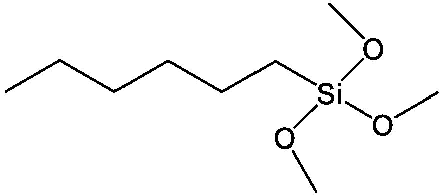
- n-Hexyltriethoxysilan 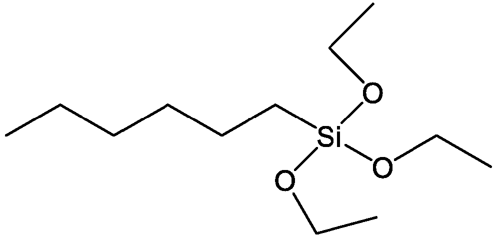
- n-Octyltrimethoxysilan 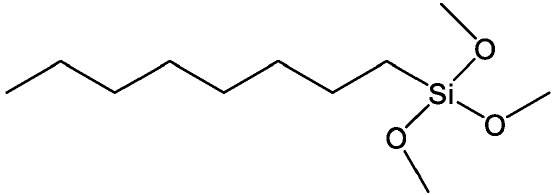
- n-Octyltriethoxysilan 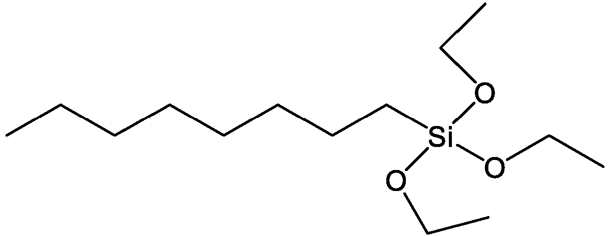
- n-Dodecyltrimethoxysilan und/oder 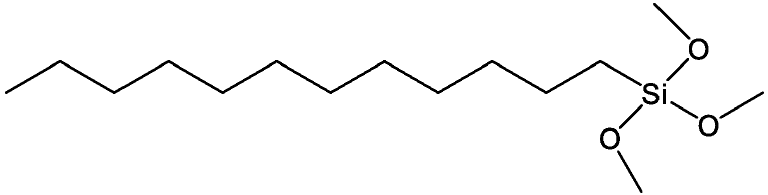
- n-Dodecyltriethoxysilan

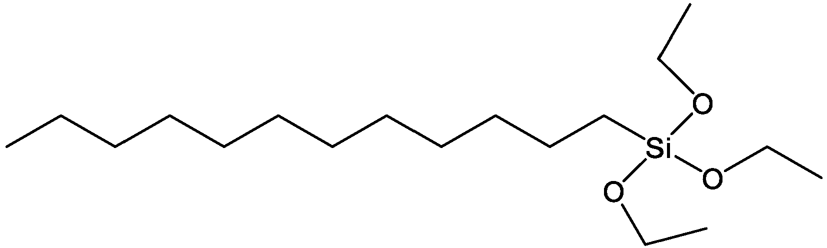

sowie Propyltrimethoxysilan, Propyltriethoxysilan, Octadecyltrimethoxysilan und/oder Octadecyltriethoxysilan.

Bei den zuvor beschriebenen organischen Siliciumverbindungen handelt es sich um reaktive Verbindungen.

Es hat sich herausgestellt, dass auf dem keratinischen Material auch dann besonders stabile und gleichmäßige Filme erhalten werden konnten, wenn das Mittel zwei strukturell voneinander verschiedene organische Siliciumverbindungen enthält.

In einer bevorzugten Ausführungsform ist ein Mittel dadurch gekennzeichnet, dass es mindestens eine organische Silicumverbindungen der Formel (I) und mindestens eine organische Silicumverbindung der Formel (IV) enthält.

In einer explizit ganz besonders bevorzugten Ausführungsform ist ein Mittel dadurch gekennzeichnet, dass es mindestens eine organische Silicumverbindungen der Formel (I) enthält, welche aus der Gruppe aus (3-Aminopropyl)triethoxysilan und (3-Aminopropyl)trimethoxysilan ausgewählt ist, und zusätzlich mindestens eine organische Silicumverbindungen der Formel (IV) enthält, welche aus der Gruppe bestehend aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan und Hexyltriethoxysilan ausgewählt ist.

In einer weiteren bevorzugten Ausführungsform ist ein Mittel dadurch gekennzeichnet, dass das Mittelbezogen auf das Gesamtgewicht des Mittels - enthält:
- 0,5 bis 5 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und (2-Dimethylaminoethyl)triethoxysilan, und
- 3,2 bis 10 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

Bereits der Zusatz geringer Wassermengen führt bei organischen Siliciumverbindungen mit mindestens einer hydrolysierbaren Gruppe zur Hydrolyse. Die Hydrolyseprodukte und/oder organischen Siliciumverbindungen mit mindestens einer Hydroxygruppe können in einer Kondensationsreaktion miteinander reagieren. Aus diesem Grund können sowohl die siliciumorganischen Verbindungen mit mindestens einer hydrolysierbaren Gruppe als auch deren Hydrolyse- und/oder Kondensationsprodukte in dem Mittel enthalten sein. Bei Verwendung von siliciumorganischen Verbindungen mit mindestens einer Hydroxylgruppe können sowohl die organischen Siliciumverbindungen mit mindestens einer Hydroxylgruppe als auch deren Kondensationsprodukte in dem Mittel enthalten sein.

Unter einem Kondensationsprodukt wird ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Im Rahmen der vorliegenden Erfindung sind Angaben in Gew.-% - falls nicht anders angegeben - immer bezogen auf das Gesamtgewicht des kosmetischen Mittels.

Als zweiten erfindungswesentlichen Bestandteil enthält das kosmetische Mittel zur Behandlung eines keratinischen Materials ein festigendes Copolymer abgeleitet aus den Monomereinheiten N-tert-Octylacrylamid, Acrylsäure und tert.-Butylaminoethylmethacrylat. Im Zuge der zu dieser Erfindung führenden Arbeiten hat sich herausgestellt, dass zur Erzielung einer besonders guten Pflegewirkung es insbesondere von Vorteil ist, wenn die organische Siliciumverbindungen, insbesondere (3-Aminopropyl)triethoxysilan, mit dem festigenden Copolymer abgeleitet aus den Monomereinheiten N-tert-Octylacrylamid, Acrylsäure und tert.-Butylaminoethylmethacrylat kombiniert wird.

Die Kombination aus der organischen Siliciumverbindung, insbesondere dem (3-Aminopropyl)triethoxysilan, mit dem festigenden Copolymer abgeleitet aus den Monomereinheiten N-tert-Octylacrylamid, Acrylsäure und tert.-Butylaminoethylmethacrylat bildet eine Schicht auf dem Haar. Diese festigenden Copolymere werden auch filmbildende Copolymere oder auch Filmbildner genannt. Dadurch wird gewährleistet, dass oxidative Haarfarben signifikant vor dem Auswaschen geschützt werden. Ferner wird die Haaroberfläche bei oxidativ geschädigtem Haar wieder hydrophobisiert, was zur Reduktion von Frizz führt. Darüber hinaus wird die Kämmbarkeit des Haares verbessert.

Festigende Copolymere, die gemäß der vorliegenden Erfindung in dem kosmetischen Mittel enthalten sein sollen, sind kommerziell erhältlich. Festigende Copolymer dieser Art sind bekannt unter der INCI-Bezeichnung "Octylacrylamide/Acrylates/Butylaminoethyl Methacrylate Copolymer (INCI)" und werden unter den Marken "Amphomer^{®}", "Amphomer^{®} HC", "Amphomer^{®} 4961", "Amphomer^{®} EDGE" und "Amphomer^{®} LV 71" in verschiedenen Abwandlungen von Akzo Nobel vertrieben wird.

Gemäß einer weiteren bevorzugten Ausführungsform der vorliegenden Erfindung enthält das kosmetisches Mittel zur Behandlung eines keratinischen Materials c) ein zweites Copolymer, das mindestens aufgebaut ist aus einem ersten Monomer, ausgewählt aus der Gruppe bestehend aus einem Methacrylsäureester, einem Acrylsäureester, Methacrylsäure und Acrylsäure, und einem zweiten Monomer, das ein vinylisches Monomer, bevorzugt Styrol, Vinylpyrrolidon oder Vinylacetat, ist, oder das ein Polyvinylpyrrolidon/Polyvinylacetat-Copolymer ist. Dieses zweite Copolymer dient als ein zusätzlicher Filmbildner.

Gemäß einer bevorzugten Ausführungsform der vorliegenden Erfindung ist das festigende Copolymer in einer Menge von 0,1 bis 10 Gew.-%, bevorzugt von 0,5 bis 8 Gew.-%, bevorzugter von 1 bis 6 Gew.-%, bezogen auf das Gesamtgewicht des kosmetischen Mittels, in dem kosmetischen Mittel enthalten.

Das Mittel zur Behandlung eines keratinischen Materials kann insbesondere ein Mittel zur Reinigung eines keratinischen Materials, ein Mittel zur Pflege eines keratinischen Materials, ein Mittel zur Pflege und Reinigung eines keratinischen Materials und/oder ein Mittel zum temporären Umformen eines keratinischen Materials umfassen.

Im Folgenden werden weitere Bestandteile der Haarbehandlungsmittel beschrieben, die neben den zuvor beschriebenen zwingenden Inhaltstoffen in den Mitteln enthalten sein können.

Es kann bevorzugt sein, dass das Mittel zur Behandlung eines keratinischen Materials ferner 0,001 bis 20 Gew.-% mindestens einer quaternären Verbindung umfasst. Dies gilt insbesondere für Mittel zur Pflege eines keratinischen Materials und für Mittel zur Pflege und Reinigung eines keratinischen Materials.

Es ist bevorzugt, dass die mindestens eine quaternäre Verbindung ausgewählt aus mindestens einer der Gruppen bestehend aus
- Amidoamine und/oder kationisierten Amidoamine und/oder
- Poly(methacryloyloxyethyltrimethylammoniumverbindungen) und/oder;
- quaternisierten Cellulose-Derivaten, insbesondere Polyquaternium 10, Polyquaternium-24, Polyquaternium-27, Polyquaternium-67, Polyquaternium-72, und/oder
- kationischen Alkylpolyglycosiden und/oder
- kationisiertem Honig und/oder
- kationischen Guar-Derivaten und/oder
- Chitosan und/oder
- polymeren Dimethyldiallylammoniumsalzen und deren Copolymeren mit Estern und Amiden von Acrylsäure und Methacrylsäure, insbesondere Polyquaternium-7 und/oder
- Copolymeren des Vinylpyrrolidons mit quaternierten Derivaten des Dialkylaminoalkylacrylats und -methacrylats, insbesondere Polyquaternium-11 und/oder
- Vinylpyrrolidon-Vinylimidazoliummethochlorid-Copolymeren, insbesondere Polyquaternium-16 und/oder
- quaterniertem Polyvinylalkohol und/oder
- Polyquaternium-74,
sowie Mischungen hiervon.

Es ist insbesondere bevorzugt, dass das Haarbehandlungsmittel ein kationisches Hompolymer, welches unter die INCI-Bezeichnung Polyquaternium-37 fällt, als quaternäre Verbindungen enthält.

Es kann bevorzugt sein, dass das Mittel zur Behandlung eines keratinischen Materials ferner eine festigende Verbindung, vorzugsweise ausgewählt aus der Gruppe bestehend aus Wachsen, synthetischen Polymeren und Mischungen daraus, umfasst.

Um den unterschiedlichen Anforderungen an Mittel zur Behandlung eines keratinischen Materials in Form eines Mittels zum temporären Umformen eines keratinischen Materials (= Stylingmittel) gerecht zu werden, sind als festigende Verbindungen bereits eine Vielzahl von synthetischen Polymeren entwickelt worden, die in dem Mittel zur Behandlung eines keratinischen Materials zur Anwendung kommen können. Alternativ oder ergänzend werden weitere synthetische Polymer und/oder Wachse als festigende Verbindungen eingesetzt. Idealerweise ergeben die synthetischen Polymere und/oder Wachse bei der Anwendung auf dem keratinischen Material einen Polymerfilm oder Film, der einerseits der Frisur einen starken Halt verleiht, andererseits aber hinreichend flexibel ist, um bei Beanspruchung nicht zu brechen.

Die synthetischen Polymere lassen sich in kationische, anionische, nichtionische und amphotere festigende Polymere unterteilen.

Geeignete synthetische Polymere umfassen beispielsweise Polymere mit den folgenden INCI-Bezeichnungen: Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/Hydroxyesters Acrylates Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis- Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer. Ebenso geeinet sind Celluloseether, wie Hydroxypropylcellulose, Hydroxyethylcellulose und Methylhydroxypropylcellulose.

Auch Homopolyacrylsäure (INCI: Carbomer), die im Handel unter dem Namen Carbopol^{®} in unterschiedlichen Ausführungen erhältlich ist, ist als festigende Verbindung geeignet.

Bevorzugt umfasst die festigende Verbindung ein Vinylpyrrolidon-haltiges Polymer. Besonders bevorzugt umfasst die festigende Verbindung ein Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI) und Mischungen daraus.

Entsprechend ist es besonders bevorzugt, dass die festigende Verbindung ein synthetisches Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI), Octylacrylamide/Acrylates/Butylaminoethyl Methacrylate Copolymer (INCI) und Mischungen daraus umfasst.

Die kosmetische Zusammensetzung kann zusätzlich oder alternativ zu einem synthetischen Polymer mindestens ein natürliches oder synthetisches Wachs, welches einen Schmelzpunkt von über 37 °C aufweist, als festigende Verbindung enthalten.

Als natürliche oder synthetische Wachse können feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie zum Beispiel Bienenwachse und andere Insektenwachse, Walrat, Schellackwachs, Wollwachs und Bürzelfett, weiterhin Mineralwachse, wie zum Beispiel Ceresin und Ozokerit oder die petrochemischen Wachse, wie zum Beispiel Petrolatum, Paraffinwachse, Microwachse aus Polyethylen oder Polypropylen und Polyethylenglycolwachse eingesetzt werden. Es kann vorteilhaft sein, hydrierte oder gehärtete Wachse einzusetzen. Weiterhin sind auch chemisch modifizierte Wachse, insbesondere die Hartwachse, zum Beispiel Montanesterwachse, Sasolwachse und hydrierte Jojobawachse, einsetzbar.

Weiterhin geeignet sind die Triglyceride gesättigter und gegebenenfalls hydroxylierter C16-30-Fettsäuren, wie zum Beispiel gehärtete Triglyceridfette (hydriertes Palmöl, hydriertes Kokosöl, hydriertes Rizinusöl), Glyceryltribehenat oder Glyceryltri-12-hydroxystearat.

Die Wachskomponenten können auch aus der Gruppe der Ester aus gesättigten, unverzweigten Alkancarbonsäuren einer Kettenlänge von 22 bis 44 C-Atomen und gesättigten, unverzweigten Alkoholen einer Kettenlänge von 22 bis 44 C-Atomen ausgewählt werden, sofern die Wachskomponente oder die Gesamtheit der Wachskomponenten bei Raumtemperatur fest sind. Auch Silikonwachse, zum Beispiel Stearyltrimethylsilan/Stearylalkohol sind gegebenenfalls vorteilhaft.

Natürliche, chemisch modifizierte und synthetische Wachse können alleine oder in Kombination eingesetzt werden. Es können somit auch mehrere Wachse eingesetzt werden. Weiterhin ist auch eine Reihe von Wachsmischungen, ggf. in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Polywax^{®} GP 200 (eine Mischung von Stearylalkohol und Polyethylenglykolstearat mit einem Schmelzpunkt von 47-51 °C; Hersteller: Croda) und "Weichceresin^{®} FL 400" (ein Vaseline/Vaselinöl/Wachs-Gemisch mit einem Schmelzpunkt von 50-54 °C; Hersteller: Parafluid Mineralölgesellschaft) sind Beispiele für einsetzbare Mischungen.

Bevorzugt ist das Wachs ausgewählt aus Carnaubawachs (INCI: Copernicia Cerifera Cera) Bienenwachs (INCI: Beeswax), Petrolatum (INCI), mikrokristallinem Wachs und insbesondere Gemischen daraus.

Bevorzugte Mischungen umfassen die Kombination von Carnaubawachs (INCI: Copernicia Cerifera Cera), Petrolatum und mikrokristallinem Wachs oder die Kombination von Bienenwachs (INCI: Beeswax) und Petrolatum.

Das Wachs oder die Wachskomponenten sollten bei 25 °C fest sein und sollen im Bereich von > 37 °C schmelzen

Das Mittel zur Behandlung eines keratinischen Materials enthält die festigende Verbindung vorzugsweise in einer Gesamtmenge von 0,5 bis 50 Gew.-%, bevorzugt 1 bis 40 Gew.-%, weiter bevorzugt 1,5 bis 30 Gew.-%, noch mehr bevorzugt 2 bis 25 Gew.-%, bezogen auf das Gesamtgewicht der kosmetischen Zusammensetzung.

Weitere geeignete Inhaltsstoffe umfassen nichtionische Polymere, anionische Polymere, (weitere) kationische Polymere, Wachse, Proteinhydrolysate, Aminosäuren, Oligopetide, Vitamine, Provitamine, Vitaminvorstufen, Betaine, Biochinone, Purin(derivate), Pflegestoffe, Pflanzenextrakte, Silikone, Esteröle, UV-Lichtschutzfilter, Strukturierungsmittel, Verdickungsmittel, Elektrolyte, pH-Stellmittel, Quellmittel, Farbstoffe, Antischuppenwirkstoffe, Komplexbildner, Trübungsmittel, Perlglanzmittel, Pigmente, Stabilisierungsmittel, Treibmittel, Antioxidantien, Parfümöle und/oder Konservierungsmittel.

In den bevorzugten Ausführungsformen 1 bis 48 werden die organischen Siliciumverbindungen der folgenden Tabelle mit dem Octylacrylamide/Acrylates/Butylaminoethyl Methacrylate Copolymer (INCI) in einem erfindungsgemäßen kosmetischen Mittel miteinander kombiniert.

| | | |
|---|---|---|
| 1 | (3-Aminopropyl)trimethoxysilan | Octylacrylamide/Acrylates/Butylaminoethyl Methacrylate Copolymer (INCI) |
| 2 | (3-Aminopropyl)triethoxysilan | Octylacrylamide/Acrylates/Butylaminoethyl Methacrylate Copolymer (INCI) |
| 3 | (2-Aminoethyl)trimethoxysilan | Octylacrylamide/Acrylates/Butylaminoethyl Methacrylate Copolymer (INCI) |
| 4 | (2-Aminoethyl)triethoxysilan | Octylacrylamide/Acrylates/Butylaminoethyl Methacrylate Copolymer (INCI) |
| 5 | (3-Dimethylaminopropyl)trimethoxysilan | Octylacrylamide/Acrylates/Butylaminoethyl Methacrylate Copolymer (INCI) |
| 6 | (3-Dimethylaminopropyl)triethoxysilan | Octylacrylamide/Acrylates/Butylaminoethyl Methacrylate Copolymer (INCI) |
| 7 | (2-Dimethylaminoethyl)trimethoxysilan | Octylacrylamide/Acrylates/Butylaminoethyl Methacrylate Copolymer (INCI) |
| 8 | (2-Dimethylaminoethyl)triethoxysilan | Octylacrylamide/Acrylates/Butylaminoethyl Methacrylate Copolymer (INCI) |
| 9 | 3-(Trimethoxysilyl)-N-[3-(trimethoxysilyl)propyl]-1-propanamin | Octylacrylamide/Acrylates/Butylaminoethyl Methacrylate Copolymer (INCI) |
| 10 | 3-(Triethoxysilyl)-N-[3-(triethoxysilyl)propyl]-1-propanamin | Octylacrylamide/Acrylates/Butylaminoethyl Methacrylate Copolymer (INCI) |
| 11 | N-Methyl-3-(trimethoxysilyl)-N-[3-(trimethoxysilyl)propyl]-1-propanamin | Octylacrylamide/Acrylates/Butylaminoethyl Methacrylate Copolymer (INCI) |
| 12 | N-Methyl-3-(triethoxysilyl)-N-[3-(triethoxysilyl)propyl]-1-propanamin | Octylacrylamide/Acrylates/Butylaminoethyl Methacrylate Copolymer (INCI) |
| 13 | 2-[Bis[3-(trimethoxysilyl)propyl]amino]-ethanol | Octylacrylamide/Acrylates/Butylaminoethyl Methacrylate Copolymer (INCI) |
| 14 | 2-[Bis[3-(triethoxysilyl)propyl]amino]-ethanol | Octylacrylamide/Acrylates/Butylaminoethyl Methacrylate Copolymer (INCI) |
| 15 | 3-(Trimethoxysilyl)-N,N-bis[3-(trimethoxysilyl)propyl]-1-propanamin | Octylacrylamide/Acrylates/Butylaminoethyl Methacrylate Copolymer (INCI) |
| 16 | 3-(Triethoxysilyl)-N,N-bis[3-(triethoxysilyl)propyl]-1-propanamin | Octylacrylamide/Acrylates/Butylaminoethyl Methacrylate Copolymer (INCI) |
| 17 | N1,N1-Bis[3-(trimethoxysilyl)propyl]-1,2-ethandiamin | Octylacrylamide/Acrylates/Butylaminoethyl Methacrylate Copolymer (INCI) |
| 18 | N 1, N 1-Bis[3-(triethoxysilyl)propyl]-1,2-ethandiamin | Octylacrylamide/Acrylates/Butylaminoethyl Methacrylate Copolymer (INCI) |
| 19 | N,N-Bis[3-(trimethoxysilyl)propyl]-2-propen-1-amin | Octylacrylamide/Acrylates/Butylaminoethyl Methacrylate Copolymer (INCI) |
| 20 | N,N-Bis[3-(triethoxysilyl)propyl]-2-propen-1-amin | Octylacrylamide/Acrylates/Butylaminoethyl Methacrylate Copolymer (INCI) |
| 21 | Methyltrimethoxysilan | Octylacrylamide/Acrylates/Butylaminoethyl Methacrylate Copolymer (INCI) |
| 22 | Methyltriethoxysilan | Octylacrylamide/Acrylates/Butylaminoethyl Methacrylate Copolymer (INCI) |
| 23 | Ethyltrimethoxysilan | Octylacrylamide/Acrylates/Butylaminoethyl Methacrylate Copolymer (INCI) |
| 24 | Ethyltriethoxysilan | Octylacrylamide/Acrylates/Butylaminoethyl Methacrylate Copolymer (INCI) |
| 25 | Octyltrimethoxysilan | Octylacrylamide/Acrylates/Butylaminoethyl Methacrylate Copolymer (INCI) |
| 26 | Octyltriethoxysilan | Octylacrylamide/Acrylates/Butylaminoethyl Methacrylate Copolymer (INCI) |
| 27 | Dodecyltrimethoxysilan | Octylacrylamide/Acrylates/Butylaminoethyl Methacrylate Copolymer (INCI) |
| 28 | Dodecyltriethoxysilan | Octylacrylamide/Acrylates/Butylaminoethyl Methacrylate Copolymer (INCI) |
| 29 | Propyltrimethoxysilan | Octylacrylamide/Acrylates/Butylaminoethyl Methacrylate Copolymer (INCI) |
| 30 | Propyltriethoxysilan | Octylacrylamide/Acrylates/Butylaminoethyl Methacrylate Copolymer (INCI) |
| 31 | Hexyltrimethoxysilan | Octylacrylamide/Acrylates/Butylaminoethyl Methacrylate Copolymer (INCI) |
| 32 | Hexyltriethoxysilan | Octylacrylamide/Acrylates/Butylaminoethyl Methacrylate Copolymer (INCI) |
| 33 | Octadecyltriethoxysilan | Octylacrylamide/Acrylates/Butylaminoethyl Methacrylate Copolymer (INCI) |
| 34 | Octadecyltriethoxysilan | Octylacrylamide/Acrylates/Butylaminoethyl Methacrylate Copolymer (INCI) |
| 35 | (3-Aminopropyl)triethoxysilan + Methyltrimethoxysilan | Octylacrylamide/Acrylates/Butylaminoethyl Methacrylate Copolymer (INCI) |
| 36 | (3-Aminopropyl)triethoxysilan + Methyltriethoxysilan | Octylacrylamide/Acrylates/Butylaminoethyl Methacrylate Copolymer (INCI) |
| 37 | (3-Aminopropyl)triethoxysilan + Ethyltrimethoxysilan | Octylacrylamide/Acrylates/Butylaminoethyl Methacrylate Copolymer (INCI) |
| 38 | (3-Aminopropyl)triethoxysilan + Ethyltriethoxysilan | Octylacrylamide/Acrylates/Butylaminoethyl Methacrylate Copolymer (INCI) |
| 39 | (3-Aminopropyl)triethoxysilan + Propyltrimethoxysilan | Octylacrylamide/Acrylates/Butylaminoethyl Methacrylate Copolymer (INCI) |
| 40 | (3-Aminopropyl)triethoxysilan + Propyltriethoxysilan | Octylacrylamide/Acrylates/Butylaminoethyl Methacrylate Copolymer (INCI) |
| 41 | (3-Aminopropyl)triethoxysilan + Hexyltrimethoxysilan | Octylacrylamide/Acrylates/Butylaminoethyl Methacrylate Copolymer (INCI) |
| 42 | (3-Aminopropyl)triethoxysilan + Hexyltriethoxysilan | Octylacrylamide/Acrylates/Butylaminoethyl Methacrylate Copolymer (INCI) |
| 43 | (3-Aminopropyl)triethoxysilan + Octyltrimethoxysilan | Octylacrylamide/Acrylates/Butylaminoethyl Methacrylate Copolymer (INCI) |
| 44 | (3-Aminopropyl)triethoxysilan + Octyltriethoxysilan | Octylacrylamide/Acrylates/Butylaminoethyl Methacrylate Copolymer (INCI) |
| 45 | (3-Aminopropyl)triethoxysilan + Dodecyltrimethoxysilan | Octylacrylamide/Acrylates/Butylaminoethyl Methacrylate Copolymer (INCI) |
| 46 | (3-Aminopropyl)triethoxysilan + Dodecyltriethoxysilan | Octylacrylamide/Acrylates/Butylaminoethyl Methacrylate Copolymer (INCI) |
| 47 | (3-Aminopropyl)triethoxysilan + Octadecyltrimethoxysilan | Octylacrylamide/Acrylates/Butylaminoethyl Methacrylate Copolymer (INCI) |
| 48 | (3-Aminopropyl)triethoxysilan + Octadecyltriethoxysilan | Octylacrylamide/Acrylates/Butylaminoethyl Methacrylate Copolymer (INCI) |

Die Wirkstoffkombination aus mindestens einer organischen Siliciumverbindung und dem festigenden Copoylmer kann bereits im Mittel zur Behandlung eines keratinischen Materials enthalten sein. In dieser Ausführungsform wird das Mittel zur Behandlung eines keratinischen Materials bereits in anwendungsbereiter Form vertrieben. Um eine möglichst lagerstabile Formulierung bereitstellen zu können, wird das Mittel selbst bevorzugt wasserarm oder wasserfrei konfektioniert.

Alternativ wird die mindestens eine organische Siliciumverbindung maximal 12 Stunden, bevorzugt maximal 6 Stunden, mehr bevorzugt maximal 3 Stunde, noch mehr bevorzugt maximal 1 Stunde vor Anwendung des Mittels zur Behandlung eines keratinischen Materials einer Basis umfassend alle Inhaltsstoffe des Mittels zur Behandlung eines keratinischen Materials mit Ausnahme der mindestens einen organischen Siliciumverbindung zugefügt.

Ferner wird alternativ die organische Siliciumverbindung und das festigende Copolymer einem kosmetischen Produkt erst kurz vor der Anwendung, d.h. 1 Minute bis 12 Stunden, bevorzugt 2 Minuten bis 6 Stunden, besonders bevorzugt 1 Minute bis 3 Stunden, insbesondere bevorzugt 1 Minute bis 1 Stunde, zugegeben.

In einer weiteren Alternative wird die organische Siliciumverbindung einer wässrigen Lösung zugegeben, welche auf das Haar appliziert wird und im zweiten Schritt wird eine wässrige Lösung oder ein kosmetisches Mittel, welches das festigende Polymer enthält, auf das Haar aufgetragen.

Beispielsweise kann der Anwender ein Mittel (α), welches die organische Siliciumverbindung(en) enthält, zunächst mit einem Mittel (β), welches die restlichen Inhaltsstoffe des Mittels zur Behandlung eines keratinischen Materials umfasst, verrühren oder verschütteln. Diese Mischung aus (α) und (β) kann der Anwender nun - entweder direkt nach ihrer Herstellung oder nach einer kurzen Reaktionszeit von 1 Minute bis 20 Minuten - auf die keratinischen Materialien applizieren. Das Mittel (β) kann Wasser enthalten, insbesondere Wasser in einer Menge > 30 Gew.-%, bezogen auf das Gesamtgewicht des Mittels zur Behandlung keratinischer Materialien.

Ein weiterer Gegenstand der vorliegenden Anmeldung ist die Verwendung eines erfindungsgemäßen kosmetischen Mittels zur Behandlung eines keratinischen Materials
zur Formgebung von keratinischem Material,
zur Pflege von keratinischem Material und/oder
zur Reduzierung und/oder Verhinderung von schädlichen Auswirkungen von Luft- und Wasserverunreinigungen auf keratinisches Material.

Bezüglich weiterer bevorzugter Ausführungsformen der Verwendung gilt mutatis mutandis das zu den kosmetischen Mitteln Gesagte.

## Patentansprüche

1. Kosmetisches Mittel zur Behandlung eines keratinischen Materials, umfassend
a) mindestens eine organische Siliciumverbindung und
b) mindestens ein festigendes Copolymer, welches zumindest aus folgenden Monomereinheiten aufgebaut ist:
- N-tert-Octylacrylamid,
- Acrylsäure, und
- tert.-Butylaminoethylmethacrylat und die INCI-Bezeichnung "Octylacrylamide/Acrylates/Butylaminoethyl Methacrylate Copolymer" besitzt,
**dadurch gekennzeichnet, dass** das Mittel zur Behandlung eines keratinischen Materialsbezogen auf das Gesamtgewicht des Mittels zur Behandlung eines keratinischen Materials - enthält:
- 0,5 bis 3,0 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und (2-Dimethylaminoethyl)triethoxysilan, und
- 3,2 bis 10,0 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Octyltrimethoxysilan, Octyltriethoxysilan, Dodecyltrimethoxysilan und Dodecyltriethoxysilan.

2. Kosmetisches Mittel zur Behandlung eines keratinischen Materials gemäß Anspruch 1, **dadurch gekennzeichnet,**
**dass** die mindestens eine organische Siliciumverbindung ferner eine Verbindung der Formel (II) enthält,
wobei in der organischen Siliciumverbindung der Formel (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} und R_{6"} unabhängig voneinander für eine C₁-C₆-Alkylgruppe stehen,
- A, A', A", A‴ und A"" unabhängig voneinander für eine lineare oder verzweigte, zweibindige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III) stehen
- (A"")-Si(R₆")_{d"}(OR₅")_{c"} (III),
- c für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

3. Kosmetisches Mittel zur Behandlung eines keratinischen Materials gemäß Anspruch 2, **dadurch gekennzeichnet,**
**dass** das Mittel zur Behandlung eines keratinischen Materials mindestens eine organische Siliciumverbindung der Formel (II) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und
- N,N-Bis[3-(triethoxysilyl)propyl]-2-Propen-1-amin.

4. Kosmetisches Mittel zur Behandlung eines keratinischen Materials gemäß einem der Ansprüche 1 bis 4, **dadurch gekennzeichnet, dass** das festigende Copolymer in einer Menge von 0,1 bis 10 Gew.-%, bevorzugt von 0,5 bis 8 Gew.-%, bevorzugter von 1 bis 6 Gew.-%, bezogen auf das Gesamtgewicht des kosmetischen Mittels, in dem kosmetischen Mittel enthalten ist.

5. Kosmetisches Mittel zur Behandlung eines keratinischen Materials gemäß einem der Ansprüche 1 bis 5, **dadurch gekennzeichnet, dass** das kosmetische Mittel
c) ein zweites Copolymer umfasst, das mindestens aufgebaut ist aus einem ersten Monomer, ausgewählt aus der Gruppe bestehend aus einem Methacrylsäureester, einem Acrylsäureester, Methacrylsäure und Acrylsäure, und einem zweiten Monomer, das ein vinylisches Monomer, bevorzugt Styrol, Vinylpyrrolidon oder Vinylacetat, ist, oder das ein Polyvinylpyrrolidon/Polyvinylacetat-Copolymer ist.

6. Verwendung eines kosmetischen Mittels zur Behandlung eines keratinischen Materials gemäß einem der Ansprüche 1 bis 5
zur Formgebung von keratinischem Material,
zur Pflege von keratinischem Material und/oder
zur Reduzierung und/oder Verhinderung von schädlichen Auswirkungen von Luft- und Wasserverunreinigungen auf keratinisches Material.

## Claims

1. A cosmetic agent for treating a keratinous material, comprising
a) at least one organosilicon compound and
b) at least one strengthening copolymer, which is made up of at least the following monomer units:
- N-tert-octylacrylamide,
- acrylic acid, and
- tert-butylaminoethyl methacrylate and the INCI name "Octylacrylamide/Acrylates/Butylaminoethyl Methacrylate Copolymer,"
**characterized in that** the agent for treating a keratinous material contains, based on the total weight of the agent for treating a keratinous material:
- 0.5 to 3.0 wt.% of at least one first organosilicon compound selected from the group consisting of (3-aminopropyl)trimethoxysilane, (3-aminopropyl)triethoxysilane, (2-aminoethyltrimethoxysilane, (2-aminoethyl)triethoxysilane, (3-dimethylaminopropyl)trimethoxysilane, (3-dimethylaminopropyl)triethoxysilane, (2-dimethylaminoethyl)trimethoxysilane and (2-dimethylaminoethyl)triethoxysilane, and
- 3.2 to 10.0 wt.% of at least one second organosilicon compound selected from the group consisting of methyltrimethoxysilane, methyltriethoxysilane, ethyltrimethoxysilane, ethyltriethoxysilane, octyltrimethoxysilane, octyltriethoxysilane, dodecyltrimethoxysilane and dodecyltriethoxysilane.

2. The cosmetic agent for treating a keratinous material according to claim 1, **characterized in that**
the at least one organosilicon compound further contains a compound of formula (II),
where, in the organosilicon compound of formula (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} and R_{6"} independently represent a C₁-C₆ alkyl group,
- A, A', A", A‴ and A"" independently represent a linear or branched, divalent C₁-C₂₀ alkylene group,
- R₇ and R₈ independently represent a hydrogen atom, a C₁-C₆ alkyl group, a hydroxy C₁-C₆ alkyl group, a C₂-C₆ alkenyl group, an amino C₁-C₆ alkyl group, or a group of formula (III)
- (Aʺʺ)-Si(R₆")_{d}"(OR₅")_{c}" (III),
- c represents an integer from 1 to 3,
- d represents the integer 3 - c,
- c' represents an integer from 1 to 3,
- d' represents the integer 3 - c',
- c" represents an integer from 1 to 3,
- d" represents the integer 3 - c",
- e represents 0 or 1,
- f represents 0 or 1,
- g represents 0 or 1,
- h represents 0 or 1,
with the proviso that at least one of the functional groups e, f, g and h is different from 0.

3. The cosmetic agent for treating a keratinous material according to claim 2, **characterized in that**
the agent for treating a keratinous material contains at least one organosilicon compound of formula (II), which is selected from the group consisting of
- 3-(trimethoxysilyl)-N-[3-(trimethoxysilyl)propyl]-1-propanamine
- 3-(triethoxysilyl)-N-[3-(triethoxysilyl)propyl]-1-propanamine
- N-methyl-3-(trimethoxysilyl)-N-[3-(trimethoxysilyl)propyl]-1-propanamine
- N-methyl-3-(triethoxysilyl)-N-[3-(triethoxysilyl)propyl]-1-propanamine
- 2-[bis[3-(trimethoxysilyl)propyl]amino]ethanol
- 2-[bis[3-(triethoxysilyl)propyl]amino]ethanol
- 3-(trimethoxysilyl)-N,N-bis[3-(trimethoxysilyl)propyl]-1-propanamine
- 3-(triethoxysilyl)-N,N-bis[3-(triethoxysilyl)propyl]-1-propanamine
- N1,N1-bis[3-(trimethoxysilyl)propyl]-1,2-ethanediamine,
- N1,N1-bis[3-(triethoxysilyl)propyl]-1,2-ethanediamine,
- N,N-bis[3-(trimethoxysilyl)propyl]-2-propen-1-amine, and
- N,N-bis[3-(triethoxysilyl)propyl]-2-propen-1-amine.

4. The cosmetic agent for treating a keratinous material according to one of claims 1 to 4, **characterized in that** the strengthening copolymer is contained in the cosmetic agent in an amount of from 0.1 to 10 wt.%, preferably from 0.5 to 8 wt.%, more preferably from 1 to 6 wt.%, based on the total weight of the cosmetic agent.

5. The cosmetic agent for treating a keratinous material according to one of claims 1 to 5, **characterized in that** the cosmetic agent
c) comprises a second copolymer which is at least made up of a first monomer selected from the group consisting of a methacrylic acid ester, an acrylic acid ester, methacrylic acid and acrylic acid, and a second monomer which is a vinylic monomer, preferably styrene, vinylpyrrolidone or vinyl acetate, or which is a polyvinylpyrrolidone/polyvinyl acetate copolymer.

6. The use of a cosmetic agent for treating a keratinous material according to one of claims 1 to 5
for shaping keratinous material,
for maintaining keratinous material and/or
for reducing and/or preventing harmful effects of air and water contaminants on keratinous material.

## Revendications

1. Agent cosmétique destiné à traiter une matière kératinique, comprenant
a) au moins un composé de silicium organique, et
b) au moins un copolymère fixateur constitué au moins des unités monomères suivantes :
- N-tert-octylacrylamide,
- acide acrylique, et
- aminoéthylméthacrylate de tert-butyle et la désignation **INCI** « Octylacrylamide/Acrylates/Butylaminoethyl Methacrylate Copolymer »,
**caractérisé en ce que** l'agent destiné à traiter une matière kératinique contient, par rapport au poids total de l'agent destiné à traiter une matière kératinique :
- 0,5 à 3,0 % en poids d'au moins un premier composé de silicium organique choisi dans le groupe constitué de (3-aminopropyl)triméthoxysilane, (3-aminopropyl)triéthoxysilane, (2-aminoéthyl)triméthoxysilane, (2-aminoéthyl)triéthoxysilane, (3-diméthylaminopropyl)triméthoxysilane, (3-diméthylaminopropyl)triéthoxysilane, (2-diméthylaminoéthyl)triméthoxysilane et (2-diméthylaminoéthyl)triéthoxysilane, et
- 3,2 à 10,0 % en poids d'au moins un second composé de silicium organique choisi dans le groupe constitué de méthyltriméthoxysilane, méthyltriéthoxysilane, éthyltriméthoxysilane, éthyltriéthoxysilane, octyltriméthoxysilane, octyltriéthoxysilane, dodécyltriméthoxysilane et dodécyltriéthoxysilane.

2. Agent cosmétique destiné à traiter une matière kératinique selon la revendication 1, **caractérisé en ce**
**que** l'au moins un composé de silicium organique contient en outre un composé de formule (II),
où, dans le composé de silicium organique de formule (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, et R_{6"} représentent, indépendamment les uns des autres, un groupe alkyle en C₁-C₆,
- A, A', A", A‴ et A"" représentent, indépendamment les uns des autres, un groupe alkylène en C₁-C₂₀ bivalent, linéaire ou ramifié,
- R₇ et R₈ représentent, indépendamment l'un de l'autre, un atome d'hydrogène, un groupe alkyle en C₁-C₆, un groupe hydroxy alkyle en C₁-C₆, un groupe alcényle en C₂-C₆, un groupe amino alkyle en C₁-C₆ ou un groupement de formule (III)
-
(A"")-Si(Re")_{d"}(OR₅")_{c"} (III),
- c représente un nombre entier allant de 1 à 3,
- d représente le nombre entier 3 - c,
- c' représente un nombre entier allant de 1 à 3,
- d' représente le nombre entier 3 - c',
- c" représente un nombre entier allant de 1 à 3,
- d" représente le nombre entier 3 - c",
- e représente 0 ou 1,
- f représente 0 ou 1,
- g représente 0 ou 1,
- h représente 0 ou 1,
à condition qu'au moins l'un des radicaux parmi e, f, g et h soit différent de 0.

3. Agent cosmétique destiné à traiter une matière kératinique selon la revendication 2, **caractérisé en ce**
**que** l'agent destiné à traiter une matière kératinique contient au moins un composé de silicium organique de formule (II) choisi dans le groupe constitué de
- 3-(triméthoxysilyl)-N-[3-(triméthoxysilyl)propyl]-1-propanamine
- 3-(triéthoxysilyl)-N-[3-(triéthoxysilyl)propyl]-1-propanamine
- N-méthyl-3-(triméthoxysilyl)-N-[3-(triméthoxysilyl)propyl]-1-propanamine
- N-méthyl-3-(triéthoxysilyl)-N-[3-(triéthoxysilyl)propyl]-1-propanamine
- 2-[bis[3-(triméthoxysilyl)propyl]amino]-éthanol
- 2-[bis[3-(triéthoxysilyl)propyl]amino]-éthanol
- 3-(triméthoxysilyl)-N,N-bis[3-(triméthoxysilyl)propyl]-1-propanamine
- 3-(triéthoxysilyl)-N,N-bis[3-(triéthoxysilyl)propyl]-1-propanamine
- N1,N1-bis[3-(triméthoxysilyl)propyl]-1,2-éthanediamine,
- N1,N1-bis[3-(triéthoxysilyl)propyl]-1,2-éthanediamine,
- N,N-bis[3-(triméthoxysilyl)propyl]-2-propén-1-amine et
- N,N-bis[3-(triéthoxysilyl)propyl]-2-propén-1-amine.

4. Agent cosmétique destiné à traiter une matière kératinique selon l'une des revendications 1 à 4, **caractérisé en ce que** le copolymère fixateur est contenu dans l'agent cosmétique en une quantité allant de 0,1 à 10 % en poids, de préférence de 0,5 à 8 % en poids, plus préférablement de 1 à 6 % en poids, par rapport au poids total de l'agent cosmétique.

5. Agent cosmétique destiné à traiter une matière kératinique selon l'une des revendications 1 à 5, **caractérisé en ce que** l'agent cosmétique c) comprend un second copolymère qui est au moins constitué d'un premier monomère choisi dans le groupe constitué d'un ester d'acide méthacrylique, ester d'acide acrylique, acide méthacrylique et acide acrylique, et d'un second monomère qui est un monomère vinylique, de préférence styrène, vinylpyrrolidone ou acétate de vinyle, ou qui est un copolymère polyvinylpyrrolidone/polyacétate de vinyle.

6. Utilisation d'un agent cosmétique destiné à traiter une matière kératinique selon l'une des revendications 1 à 5
pour la mise en forme de la matière kératinique,
pour le soin de la matière kératinique et/ou
pour la réduction et/ou la prévention d'effets nocifs de la pollution de l'air et de l'eau sur la matière kératinique.
